# FASCICULE DE BREVET EUROPEEN

(11) **EP 1 173 273 B1**
(45) Date de publication et mention de la délivrance du brevet: **03.11.2004**
(21) Numéro de dépôt: 00912714.3
(22) Date de dépôt: 17.03.2000
(51) Int. Cl.: B01F 17/00

(54) **NOUVELLES EMULSIONS EAU-DANS-HUILE STABLES CONTENANT UN EMULSIONNANT A BASE D'OLEYL- ET/OU D'ISOSTEARYL-GLYCOSIDE**
OLEYL- UND/ODER ISOSTEARYL-GLYCOSID ENTHALTENDE STABILE NEUE WASSER-IN-ÖL-EMULSIONEN
NOVEL STABLE WATER-IN-OIL EMULSIONS CONTAINING AN EMULSIFIER BASED ON OLEYL- AND/OR ISOSTEARYL-GLYCOSIDE

(30) Priorité: 19.03.1999 FR 9903429
(43) Date de publication de la demande: 23.01.2002
(73) Titulaire: SOCIETE D'EXPLOITATION DE PRODUITS POUR LES INDUSTRIES CHIMIQUES, S.E.P.P.I.C., 75321 Paris Cédex 07 (FR)
(72) Inventeur: MILIUS, Alain, F-06000 Nice (FR); ROSO, Alicia, F-81710 Saix (FR); MICHEL, Nelly, F-94700 Maisons Alfort (FR)
(74) Mandataire: Conan, Philippe Claude
(86) Numéro de dépôt international: PCT/FR2000/000656
(87) Numéro de publication internationale: WO 2000/056438

(56) Documents cités:
- WO-A-98/22207

## Description

La présente invention a pour objet de nouvelles émulsions eau-dans-huile stables contenant un émulsionnant à base d'oléyl- et/ou d'isostéarylglycoside.

L'invention trouve notamment application dans le domaine cosmétique.

Les alkylglycosides ou alkylpolyglycosides (APG) sont des composés tensioactifs non ioniques bien connus qui peuvent être utilisés seuls, ou en association avec d'autres tensioactifs, dans une large gamme d'applications industrielles et notamment dans le domaine cosmétique.

Les alkylpolyglycosides ont d'abord été utilisés comme agents moussants et dans cette application, ceux dont la chaîne alkyle comporte de 8 à 14 atomes de carbone se sont avérés particulièrement intéressants.

Plus récemment, les alkylpolyglycosides ont été utilisés comme émulsionnants, et dans cette application, ceux dont la chaîne alkyle comporte de 16 à 18 atomes de carbone se sont avérés particulièrement intéressants.

La demande de brevet WO 92/06778, au nom de la demanderesse, décrit pour la première fois l'utilisation de mélanges d'alkylpolyglycosides et d'alcools gras en tant qu'agents auto-émulsionnants.

Par "auto-émulsionnant", on désigne tout agent ou composition capable de former une émulsion stable avec une phase aqueuse, pratiquement sans apport d'énergie, par exemple par dispersion dans la phase aqueuse par agitation mécanique lente.

Plus précisément, les mélanges décrits dans ce document antérieur comprennent :
- de 60 à 90 % en poids d'au moins un alcool gras ayant de 12 à 22 atomes de carbone, et de préférence de 16 à 18 atomes de carbone ; et
- de 10 à 40 % en poids d'un alkylpolyglycoside, dont la partie alkyle est de préférence identique à celle de l'alcool gras.

Les compositions auto-émulsionnables décrites dans la demande précitée sont commercialisées sous la dénomination Montanov® 68 et comportent un mélange d'alkylpolyglycosides dont les chaînes grasses comprennent 16 et 18 atomes de carbone, ainsi qu'un mélange d'alcools gras de même longueur de chaînes grasses.

A l'exemple 3.5 de ce document de l'état de la technique, on a décrit une émulsion comprenant :
- 5 % en poids d'une phase grasse constituée de cétylstéaryloctanoate,
- 10 % en poids d'une composition auto-émulsionnante à base d'oléylglycoside et d'alcool oléique.

Cette émulsion est de type classique huile-dans-eau.

Postérieurement à la demande de brevet WO 92/06778, de nombreux documents brevets ont décrit l'utilisation de compositions émulsionnantes ou auto-émulsionnantes à base d'alkylpolyglycosides et d'alcools gras pour la préparation d'émulsions.

Il est envisagé dans ces documents d'utiliser des alkylglycosides de nature variée, dont la partie alkyle peut être ramifiée et/ou insaturée et présenter de 4 à 54 atomes de carbone.

Si certains de ces documents envisagent également explicitement l'utilisation d'oléylglycoside ou d'isostéarylglycoside, comme par exemple les documents WO 97/02091 ou WO 97/18033, c'est toujours dans des conditions conduisant à la réalisation d'émulsions classiques huile-dans-eau.

Par conséquent, aucun de ces brevets antérieurs ne vise spécifiquement la préparation d'émulsions eau-dans-huile qui présentent cependant un intérêt considérable, notamment dans le domaine cosmétique.

Il a été découvert, et ceci constitue le fondement de la présente invention, que des compositions à base d'oléyl- et/ou d'isostéaryl-glycoside associées à des mélanges d'alcool oléique et d'alcool isostéarylique peuvent être utilisées, dans certaines conditions, comme émulsionnants pour la préparation d'émulsions eau-dans-huile.

Cette découverte est tout à fait inattendue, dans la mesure où il a par ailleurs été constaté que des compositions à base d'alkylglycosides et d'alcools gras correspondants, comme par exemple un isooctadécylglycoside, ou un alkylglycoside obtenu à partir d'un alcool de Guerbet ayant 18 atomes de carbone ne permettent pas d'obtenir des émulsions eau-dans-huile.

Les nouvelles émulsions eau-dans-huile conformes à la présente invention contiennent :
- 10 à 70 % en poids d'une phase aqueuse ;
- 25 à 60 % en poids d'une phase grasse ; et
- 5 à 15 % en poids d'un émulsionnant comprenant :
   - 10 à 90 % en poids, de préférence 10 à 50 % en poids et de préférence encore de 10 à 40 % en poids d'un mélange en toute proportion d'un oléylglycoside ayant un degré de polymérisation compris entre 1 et 3 et d'un isostéarylglycoside ayant un degré de polymérisation compris entre 1 et 3 ; et
   - 90 à 10 % en poids, de préférence 90 à 50 % en poids, et de préférence encore de 90 à 60 % en poids d'un mélange en toute proportion d'alcool oléique et d'alcool isostérarylique ;
   et éventuellement :
- 0 à 10 % en poids d'un co-émulsionnant ;
- 0 à 5 % en poids d'un stabilisant.

Des émulsions eau-dans-huile stables particulièrement préférées dans le cadre de la présente invention contiennent :
- 10 à 25 % en poids d'un isostéarylglycoside ayant un degré de polymérisation compris entre 1 et 3 ; et
- 90 à 75 % en poids d'un alcool isostéarylique.

Dans le cadre de la présente description, on entend désigner par les expressions "oléylglycoside ayant un degré de polymérisation compris entre 1 et 3" et "isostéarylglycoside ayant un degré de polymérisation compris entre 1 et 3", des composés de formule :

RO(G)ₓ

dans laquelle :
R représente respectivement un radical oléyle et isostéaryle et
x représente un nombre compris entre 1 et 3 ;
G représente un reste de glycopyranose ou glycofuranose réducteur et de préférence un reste de glucose.

Avantageusement, le degré de polymérisation de ces alkylglycosides représenté par x est compris entre 1,05 et 2,5 ; et de préférence encore, entre 1,1 et 2.

Les alkylglycosides utilisés dans le cadre de la présente invention en tant qu'émulsionnants sont des composés dont les radicaux alkyles sont définis comme indiqué précédemment.

Cependant, ces composés ne sont pas toujours purs.

Ils peuvent en effet contenir en outre des proportions mineures de composés de même nature dont les radicaux alkyles comportent une chaîne plus longue et/ou plus courte, de tels composés provenant notamment des alcools gras généralement d'origine naturelle ou synthétique utilisés comme matière de départ pour la synthèse de ces composés.

Par "proportion mineure", on entend une quantité cumulée maximale "d'impuretés" de 5 % en poids, et de préférence de 1 % en poids rapporté au poids total des alkylglycosides précités.

Les compositions à base d'isostéarylglycoside et d'alcool isostéarylique se sont avérées particulièrement intéressantes, en tant qu'émulsionnant pour la préparation d'émulsions eau-dans-huile stables.

On comprend ainsi que l'expression "mélange en toute proportion" vise également à couvrir l'utilisation d'oléylglycoside ou d'isostéarylglycoside seuls, c'est-à-dire en fait des mélanges comprenant de 0 à 100% en poids de chacun des deux constituants précités.

Les compositions à base d'oléyl- et/ou d'isostéarylglycoside et d'alcool oléique et/ou d'alcool isostéarylique utilisées comme émulsionnant dans le cadre de la présente invention peuvent être préparées par simple mélange de leurs constituants en des proportions prédéterminées souhaitées.

A l'échelle industrielle, on les préparera de préférence selon l'une des deux voies classiquement utilisées pour la synthèse des alkylpolyglycosides, et notamment par réaction en milieu acide entre le, ou les alcools gras correspondants, et un saccharide disposant d'un groupement OH anomérique tel que le glucose ou le dextrose.

De telles voies de synthèse sont bien connues, et ont été largement décrites dans la littérature et en particulier dans les documents de l'état de la technique cité en introduction.

Le cas échéant, cette synthèse pourra être complétée par des opérations de neutralisation, de filtration, de distillation ou d'extraction partielle de l'alcool gras en excès ou de décoloration.

D'une façon générale, les nouvelles émulsions conformes à la présente invention contiennent de 25 à 60% en poids d'une phase grasse ou huileuse.

Cette phase grasse peut être constituée par une ou plusieurs huiles choisies parmi les huiles suivantes :
- les huiles d'origine végétale, telles que l'huile d'amandes douces, l'huile de coprah, l'huile de ricin, l'huile de jojoba, l'huile d'olive, l'huile de colza, l'huile d'arachide, l'huile de tournesol, l'huile de germes de blé, l'huile de germes de maïs, l'huile de soja, l'huile de coton, l'huile de luzerne, l'huile de pavot, l'huile de potiron, l'huile d'onagre, l'huile de millet, l'huile d'orge, l'huile de seigle, l'huile de carthame, l'huile de bancoulier, l'huile de passiflore, l'huile de noisette, l'huile de palme, le beurre de karité, l'huile de noyau d'abricot, l'huile de calophyllum, l'huile de sysymbrium, l'huile d'avocat, l'huile de calendula ;
- les huiles végétales modifiées telles que les produits connus sous les dénominations INCI, Apricot Kernel Oil PEG-6 esters et Olive Oil PEG-6 esters ;
- les huiles d'origine naturelle, telles que le perhydrosqualène, le squalène ;
- les huiles minérales, telles que l'huile de paraffine ou huile de vaseline, et les huiles minérales, notamment issues de coupes pétrolières, telles que les isoparaffines, ayant un point d'ébullition compris entre 300 et 400°C ;
- les huiles synthétiques, notamment les esters d'acides gras tels que le myristate de butyle, le myristate de propyle, le myristate de cétyle, le palmitate d'isopropyle, le stéarate de butyle, le stéarate d'hexadécyle, le stéarate d'isopropyle, le stéarate d'octyle, le stéarate d'isocétyle, l'oléate dodécyle, le laurate d'hexyle, le dicaprylate de propylèneglycol, les esters dérivés d'acide lanolique, tels que le lanolate d'isopropyle, le lanolate d'isocétyle, les monoglycérides, les triglycérides comme le triheptanoate de glycérol, les alkylbenzoates, les isoparaffines, les polyalphaoléfines, les polyoléfines, comme le polyisobutène les isoalcanes de synthèse comme l'isohexadecane, l'isododécane, les huiles perfluorées et les huiles de silicone. Parmi ces dernières, on peut plus particulièrement citer les diméthylpolysiloxanes, méthylphénylpolysiloxanes, les silicones modifiés par des amines, les silicones modifiés par des acides gras, les silicones modifiés par des alcools, les silicones modifiés par des alcools et des acides gras, des silicones modifiés par des groupements polyéther, des silicones époxy modifiés, des silicones modifiés par des groupements fluorés, des silicones cycliques et des silicones modifiés par des groupements alkyles.

Cette phase grasse peut également contenir un ou plusieurs composés choisis parmi les acides gras, les alcools gras, les cires d'origine naturelle ou synthétique, et plus généralement encore tout corps gras d'origine végétale, animale ou synthétique.

D'une façon générale, les émulsions conformes à la présente invention contiennent de 10 à 70 % en poids d'une phase hydrophile essentiellement constituée d'eau et éventuellement d'un solvant hydrophile, comme par exemple la glycérine.

Les émulsions conformes à la présente invention peuvent également contenir, de façon optionnelle, jusqu'à 10 % en poids d'un co-émulsionnant et jusqu'à 10 % en poids d'un stabilisant.

Parmi les agents co-émulsionnants susceptibles d'être utilisés dans le cadre de la présente invention, on citera notamment les lipoaminoacides et leurs sels, les lipopeptides et leurs sels, les émulsionnants siliconés non-ioniques et anioniques, les esters de sorbitan, les esters de polyglycérol, l'huile de ricin hydrogénée éthoxylée, le stéarate de glycérol, les polyhydroxystéarates de polyglycol comme par exemple le produit dénommé HYPERMER®, les huiles végétales éthoxylées, les esters méthyliques d'huiles végétales éthoxylés, les esters de sorbitan éthoxylés comme par exemple les produits commercialisés sous la dénomination POLYSORBATE 81®, POLYSORBATE 61® et POLYSORBATE 21® ; les acylats de protéines faiblement éthoxylés (de 1 à 3 groupements OE) ; les émulsionnants cationiques comme les aminoxydes, le quaternium 82 et les tensio-actifs décrits dans la demande de brevet WO 96/00719 et principalement ceux dont la chaîne grasse comprend au moins 16 atomes de carbone ; les esters de sucrose, les esters de méthylglucoside ethoxylés ou non ; les acides gras éthoxylés ; les alcools gras éthoxylés ; les émulsionnants anioniques comme le décylphosphate ou le cétéarylsulfate.

Avantageusement, en tant que co-émulsionnant, on utilisera dans le cadre de la présente invention, un ester de sorbitan comme en particulier un oléate de sorbitan.

Parmi les agents stabilisants susceptibles d'être utilisés dans le cadre de la présente invention, on peut citer l'huile de ricin hydrogénée, les cires végétales comme par exemple la cire d'abeille et la cire de carnauba, l'acide stéarique, le polyoxystéarate d'aluminium, tel que par exemple le produit commercialisé sous la dénomination MANALOX® , le stéarate de magnésium, le stéarate d'aluminium, les silices hydrophobes, les copolymères polyéthylèneglycol-alkylglycol, les polymères tels que les produits commercialisés sous la dénomination KRATON® par la société SHELL CHEMICALS, les cires minérales comme l'ozokérite.

Avantageusement, l'agent stabilisant sera un copolymère polyéthylèneglycol-dodécylglycol notamment le PEG-45 dodécylglycol copolymère tel que le produit commercialisé sous la dénomination ELFACOS ST 9 ®, par la société AKZO NOBEL.

Dans un mode de réalisation particulièrement préféré, on utilisera en tant que co-émulsionnant et stabilisant un mélange de cire d'abeille, d'huile de ricin hydrogénée, d'acide stéarique et d'oléate de sorbitan, comme en particulier le produit commercialisé sous la dénomination MONTANE® 481, par la société SEPPIC.

Les émulsions eau-dans-huile stables, conformes à la présente invention, pourront être préparées par simple dispersion, à une température comprise entre 15°C et 85°C, de la phase aqueuse dans la phase grasse, en présence de l'émulsionnant, et éventuellement du co-émulsionnant et/ou du stabilisant, ces derniers étant généralement présents au sein de la phase grasse.

D'une façon connue en soi, ces émulsions peuvent en outre comprendre un ou plusieurs composés choisis parmi les humectants, comme par exemple la glycérine, les conservateurs comme par exemple les produits connus sous la dénomination SEPICIDE® , les colorants, les parfums, les actifs cosmétiques, les filtres solaires minéraux ou organiques, les charges minérales comme les oxydes de fer, oxydes de titane et le talc, les charges synthétiques comme les nylons et les micropearls et les extraits de plantes.

Ces composés pourront être introduits dans la phase aqueuse ou dans la phase grasse, selon leur affinité pour ces phases, soit au cours de la phase de dispersion précitée, soit en ce qui concerne les composés sensibles à la température, postérieurement au cours de la phase de refroidissement dans le cas où la dispersion est réalisée à chaud.

Selon un second aspect, la présente invention a pour objet l'utilisation d'une composition comprenant :
- 10 à 90 % en poids, de préférence 10 à 50 % en poids et de préférence encore de 10 à 40 % en poids d'un mélange en toute proportion d'un oléylglycoside ayant un degré de polymérisation compris entre 1 et 3 et d'un isostéarylglycoside ayant un degré de polymérisation compris entre 1 et 3 ; et
- 90 à 10 % en poids, de préférence 90 à 50 % en poids, et de préférence encore de 90 à 60 % en poids d'un mélange en toute proportion d'alcool oléique et d'alcool isostérarylique ;
comme émulsionnant destiné à la préparation d'une émulsion eau-dans-huile stable.

L'invention sera illustrée plus en détail par les exemples suivants, donnés uniquement à titre illustratif.

### EXEMPLE 1

### Procédé de préparation d'une composition à base d'isostéarylglycoside et d'alcool isostéarylique utile comme émulsionnant pour la préparation d'une émulsion eau-dans-huile selon l'invention.

On introduit dans un réacteur polyvalent de l'alcool isostéarylique (produit commercialisé par la Société UNICHEMA sous la dénomination PRISORINE®3515, ou par la société HENKEL sous la dénomination SPEZIOL C18 ISO.

On introduit également dans le réacteur du glucose, de sorte que le rapport molaire entre l'alcool isostéarylique et le glucose soit de : 6/1.

On fait ensuite réagir le glucose avec l'alcool gras pendant 6 heures à une température d'environ 100°C, en présence d'un catalyseur acide sous vide partiel.

Après réaction, on neutralise le catalyseur au moyen d'une base.

La composition obtenue présente les caractéristiques analytiques suivantes :
- aspect : liquide

### EXEMPLE 2

### Procédé de préparation d'une composition à base d'oléylglycoside et d'alcool oléique utile comme émulsionnant pour la préparation d'une émulsion eau-dans-huile selon l'invention.

La composition de l'exemple 2 a été réalisée en suivant le protocole expérimental décrit à l'exemple 1, mais en remplacant l'alcool isostéarylique par de l'alcool oléique.

Les caractéristiques analytiques du produit ainsi obtenu, sont les suivantes :
- aspect : liquide

### EXEMPLES COMPARATIFS 1 à 6

On a préparé divers émulsionnants à base d'alkylpolyglycosides et d'alcools gras afin de mettre en évidence les propriétés particulières des mélanges à base d'oléyl- et/ou d'isostéarylglucoside utilisés dans le cadre de la présente invention.

Le produit de l'exemple comparatif 1 a été préparé en suivant le protocole expérimental de l'exemple 1, de façon à obtenir une composition à base de cétéarylglycoside et d'alcool cétéarylique telle que celle définie aux exemples R3 et R4 du document WO 97/18033.

Le produit de l'exemple comparatif 2 a été préparé en suivant le protocole expérimental décrit à l'exemple 1 du document WO 92/06778.

Le produit de l'exemple comparatif 3 été préparé en suivant le protocole expérimental de l'exemple 4 du document WO 97/02091.

Le produit de l'exemple comparatif 4 a été préparé en suivant le protocole expérimental de l'exemple de comparaison F du document WO 98/22207.

Le produit de l'exemple comparatif 5 a été préparé en suivant le protocole expérimental décrit à l'exemple 1 et en remplaçant l'alcool isostéarylique par de l'alcool isooctadécylique (alcool oxo C18 commercialisé par la société HOECHST).

Les caractéristiques analytiques du produit ainsi obtenu sont les suivantes :
- aspect : liquide

Le produit de l'exemple comparatif 6 a été préparé en suivant le protocole expérimental de l'exemple 1 en remplaçant l'alcool isostéarylique par un alcool de Guerbet en C 18, commercialisé par la Société Comdea sous la dénomination ISOFOL®18T.

Les caractéristiques analytiques du produit ainsi obtenu sont les suivantes :
- aspect : liquide

On a regroupé dans le tableau 1 ci-après les différentes compositions qualitatives et quantitatives des produits des exemples 1 et 2 et des exemples comparatifs 1 à 6.

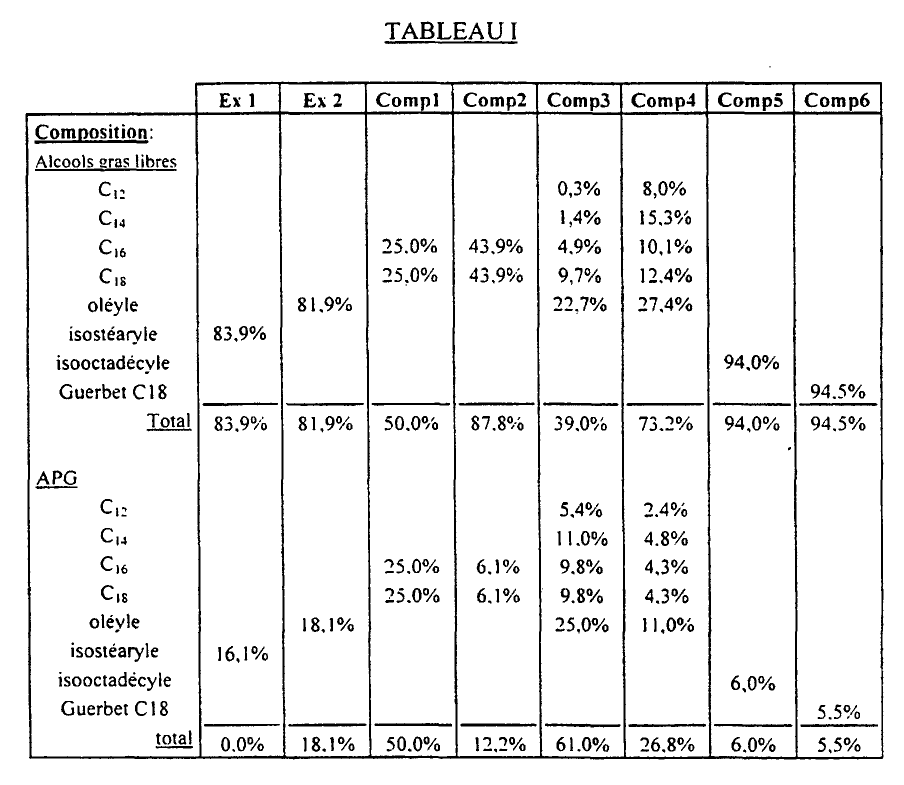

### EXEMPLE 3

### Mise en évidence des propriétés remarquables des compositions à base d'oléyl- et/ou d'isostéarylglycoside en tant qu'émulsionnant pour la préparation d'émulsions eau-dans-huile stables.

On a réalisé des émulsions en utilisant en tant qu'émulsionnant les produits des exemples 1 et 2 et des exemples comparatifs 1 à 6 dans les conditions décrites dans l'état de la technique, et en particulier à l'exemple 3 du document WO 92/06778.

Dans les conditions expérimentales utilisées (jusqu'à 17 % en poids de phase grasse et 5 % en poids d'émulsionnant), tous les produits des exemples ainsi que des exemples comparatifs ont conduit à l'obtention d'émulsions classiques huile-dans-eau (détermination par la méthode de la goutte).

On a ensuite réalisé de nouvelles émulsions en utilisant une phase grasse à base d'huile de paraffine en une quantité en poids y %, l'émulsionnant étant utilisé en une quantité de x % en poids.

Les émulsions ainsi réalisées présentent les compositions suivantes :
- système émulsionnant : x%
- stabilisant (PEG 45 dodecyl glycol copolymer = ELFACOS ST 9 de AKZO):2%
- phase grasse : y%
- MgSO4 : 0,7 %
- glycérine : 5 %
- conservateur : qsp
- eau : qsp 100 %

Cette étude a permis de mettre en évidence le fait que seuls les émulsionnants des exemples 1 et 2 permettent d'obtenir dans les conditions expérimentales mentionnées des émulsions de type eau-dans-huile quand x est supérieur ou égal à 3 % et y est supérieur ou égal à 20 %, tous les émulsionnants des exemples comparatifs 1 à 6 conduisant à des émulsions de type huile-dans-eau.

On voit donc la spécificité des émulsionnants à base d'oléyl- et/ou isostéarylglycoside par rapport à des alkylglycosides de nature chimique voisine, vis-à-vis de l'aptitude à former des émulsions eau-dans-huile.

On donnera maintenant quelques exemples d'émulsions eau-dans-huile selon l'invention dans le domaine d'application de la cosmétique.

### EXEMPLES D'EMULSIONS EAU-DANS-HUILE STABLES SELON L'INVENTION.

Ces émulsions ont été préparées selon le mode opératoire suivant :
a) on chauffe à 70°-75°C une composition "A" (phase grasse) contenant un émulsionnant à base d'oléyl- et/ou d'isostéarylglycoside (produit de l'exemple 1 ou de l'exemple 2) ;
b) on chauffe à 70°-75°C une composition "B" (phase aqueuse) ;
c) on forme une émulsion en mélangeant la phase aqueuse "B" dans la phase grasse "A" au moyen d'un agitateur de type SILVERSON 4'-4000 rpm ;
d) on refroidit sous ancre ;
e) on ajoute une composition "C" (conservateur, parfum, colorants etc...) vers 40°C.

| CREME BEBE E/H | |
|---|---|
| A | |
| Produit de l'exemple 1 | 8 % |
| huile de paraffine | 35 % |
| huile d'amandes douces | 5 % |
| cire d'abeille | 3 % |
| huile de ricin | 2 % |
| oxyde de zinc | 10 % |
| SEPICALM®VG | 1 % |

| B | |
|---|---|
| eau | QSP 100 |
| glycérine | 5 % |
| MgSO4 | 0,7% |

| C | |
|---|---|
| Carotène | 0,05 % |
| dl alpha tocopherol | 0,05 % |
| parfum | 0,2 % |
| SEPICIDE®HB | 0,5 % |
| SEPICIDE®Cl | 0,2 % |

| CREME E/H | |
|---|---|
| A | |
| Produit de l'exemple 2 | 5 % |
| Squalane | 30 % |
| MONTANE®481 | 2% |

| B | |
|---|---|
| eau | QSP 100 |
| glycérine | 5 % |
| SEPICALM®S | 21 % |

| C | |
|---|---|
| dl alpha tocopherol | 0,05 % |
| parfum | 0,2% |
| SEPICIDE®HB | 0,3 % |
| SEPICIDE®CI | 0,2 % |

| LAIT HYDRATANT E/H VAPORISABLE | |
|---|---|
| A | |
| Produit de l'exemple 1 | 8 % |
| ELFACOS ST 9 | 2 % |
| huile minérale | 40 % |
| SEPICIDE® HB | 1 % |

| B | |
|---|---|
| eau | QSP 100 |
| glycérine | 5 % |
| MgCl₂ | 0,7% |

| CREME E/H | |
|---|---|
| A | |
| Produit de l'exemple 1 | 3 % |
| ELFACOS® ST 9 | 2 % |
| MONTANE® 481 | 6 % |
| huile minérale | 30 % |
| SEPICIDE® HB | 1 % |

| B | |
|---|---|
| eau | QSP 100 |
| glycérine | 5 % |
| MgCl₂ | 0,7 % |

## Revendications

1. Emulsions "eau-dans-huile" stables, **caractérisées en ce qu'**elles contiennent :
- 10 à 70 % en poids d'une phase aqueuse ;
- 25 à 60 % en poids d'une phase grasse ; et
- 5 à 15 % en poids d'un émulsionnant comprenant :
• 10 à 90 % en poids, de préférence 10 à 50 % en poids et de préférence encore de 10 à 40 % en poids d'un mélange en toute proportion d'un oléylglycoside ayant un degré de polymérisation compris entre 1 et 3 et d'un isostéarylglycoside ayant un degré de polymérisation compris entre 1 et 3 ; et
• 90 à 10 % en poids, de préférence 90 à 50 % en poids, et de préférence encore de 90 à 60 % en poids d'un mélange en toute proportion d'alcool oléique et d'alcool isostérarylique ;
et éventuellement :
- 0 à 10 % en poids d'un co-émulsionnant ;
- 0 à 5 % en poids d'un stabilisant.

2. Emulsions selon la revendication 1, **caractérisées en ce que** l'émulsionnant précité comprend :
• 10 à 25 % en poids d'un isostéarylglycoside ayant un degré de polymérisation compris entre 1 et 3 ; et
• 90 à 75 % en poids d'un alcool isostéarylique.

3. Emulsions selon la revendication 1, **caractérisées en ce que** l'émulsionnant précité comprend :
• 10 à 25 % en poids d'un oléylglycoside ayant un degré de polymérisation compris entre 1 et 3 ; et
• 90 à 75 % en poids d'un alcool oléique.

4. Emulsions selon l'une quelconque des revendications 1 à 3, **caractérisées en ce que** la phase grasse précitée est constituée d'une ou plusieurs huiles choisies parmi les huiles minérales, notamment les huiles de paraffine.

5. Emulsions selon l'une quelconque des revendications 1 à 4 **caractérisées en ce que** le co-émulsionnant précité est constitué par un ester de sorbitan, de préférence un oléate de sorbitan.

6. Emulsions selon l'une quelconque des revendications 1 à 5, **caractérisées en ce que** le stabilisant précité est choisi parmi un copolymère polyéthylène glycol-dodécylglycol tel que le produit commercialisé sous la dénomination ELFACOS ST 9 ® ; la cire d'abeille, l'acide stéarique ou l'huile de ricin hydrogénée.

7. Emulsions selon l'une quelconque des revendications 1 à 6, **caractérisées en ce qu'**elles contiennent, en tant que co-émulsionnant et stabilisant un mélange de cire d'abeille, d'huile de ricin hydrogénée, d'acide stéarique et d'oléate de sorbitan, comme en particulier le produit commercialisé sous la dénomination MONTANE® 481.

8. Utilisation d'une composition constituée de :
• 10 à 90 % en poids, de préférence 10 à 50 % en poids et de préférence encore de 10 à 40 % en poids d'un mélange en toute proportion d'un oléylglycoside ayant un degré de polymérisation compris entre 1 et 3 et d'un isostéarylglycoside ayant un degré de polymérisation compris entre 1 et 3 ; et
• 90 à 10 % en poids, de préférence 90 à 50 % en poids, et de préférence encore de 90 à 60 % en poids d'un mélange en toute proportion d'alcool oléique et d'alcool isostérarylique ;
comme émulsionnant destiné à la préparation d'une émulsion eau-dans-huile stable.

9. Utilisation selon la revendication 8 d'une composition constituée de:
• 10 à 25 % en poids d'un isostéarylglycoside ayant un degré de polymérisation compris entre 1 et 3 ; et
• 90 à 75 % en poids d'un alcool isostéarylique,
comme émulsionnant destiné à la préparation d'une émulsion eau-dans-huile stable.

10. Utilisation selon la revendication 8 d'une composition constituée de:
• 10 à 25 % en poids d'un oléylglycoside ayant un degré de polymérisation compris entre 1 et 3 ; et
• 90 à 75 % en poids d'un alcool oléique,
comme émulsionnant destiné à la préparation d'une émulsion eau-dans-huile stable.

## Claims

1. Stable water-in-oil emulsions, **characterized in that** they comprise:
- 10 to 70% by weight of an aqueous phase;
- 25 to 60% by weight of a fatty phase; and
- 5 to 15% by weight of an emulsifier comprising:
• 10 to 90% by weight, preferably 10 to 50% by weight and more preferably from 10 to 40% by weight of a mixture in any proportion of an oleyl glycoside having a degree of polymerization of between 1 and 3 and an isostearyl glycoside having a degree of polymerization of between 1 and 3; and
• 90 to 10% by weight, preferably 90 to 50% by weight and more preferably from 90 to 60% by weight of a mixture in any proportion of oleyl alcohol and isostearyl alcohol;
and optionally:
- 0 to 10% by weight of a co-emulsifier;
- 0 to 5% by weight of a stabilizer.

2. Emulsions according to Claim 1, **characterized in that** the aforementioned emulsifier comprises:
• 10 to 25% by weight of an isostearyl glycoside having a degree of polymerization of between 1 and 3; and
• 90 to 75% by weight of an isostearyl alcohol.

3. Emulsions according to Claim 1, **characterized in that** the aforementioned emulsifier comprises:
• 10 to 25% by weight of an oleyl glycoside having a degree of polymerization of between 1 and 3; and
• 90 to 75% by weight of an oleyl alcohol.

4. Emulsions according to any one of Claims 1 to 3, **characterized in that** the aforementioned fatty phase is composed of one or more oils selected from mineral oils, especially paraffin oils.

5. Emulsions according to any one of Claims 1 to 4, **characterized in that** the aforementioned co-emulsifier is composed of a sorbitan ester, preferably a sorbitan oleate.

6. Emulsions according to any one of Claims 1 to 5, **characterized in that** the aforementioned stabilizer is selected from a polyethylene glycol-dodecyl glycol copolymer such as the product sold under the name Elfacos ST 9® ; beeswax, stearic acid and hydrogenated castor oil.

7. Emulsions according to any one of Claims 1 to 6, **characterized in that** they comprise, as co-emulsifier and stabilizer, a mixture of beeswax, hydrogenated castor oil, stearic acid and sorbitan oleate, such as in particular the product sold under the name Montane® 481.

8. Use of a composition consisting of:
• 10 to 90% by weight, preferably 10 to 50% by weight and more preferably from 10 to 40% by weight of a mixture in any proportion of an oleyl glycoside having a degree of polymerization of between 1 and 3 and an isostearyl glycoside having a degree of polymerization of between 1 and 3; and
• 90 to 10% by weight, preferably 90 to 50% by weight and more preferably from 90 to 60% by weight of a mixture in any proportion of oleyl alcohol and isostearyl alcohol;
as an emulsifier intended for preparing a stable water-in-oil emulsion.

9. Use according to Claim 8 of a composition consisting of:
• 10 to 25% by weight of an isostearyl glycoside having a degree of polymerization of between 1 and 3; and
• 90 to 75% by weight of an isostearyl alcohol,
as an emulsifier intended for preparing a stable water-in-oil emulsion.

10. Use according to Claim 8 of a composition consisting of:
• 10 to 25% by weight of an oleyl glycoside having a degree of polymerization of between 1 and 3; and
• 90 to 75% by weight of an oleyl alcohol,
as an emulsifier intended for preparing a stable water-in-oil emulsion.

## Patentansprüche

1. Stabile "Wasser-in-Öl"-Emulsionen, **dadurch gekennzeichnet, dass** sie folgendes enthalten:
- 10 bis 70 Gew.-% einer wässrigen Phase,
- 25 bis 60 Gew.-% einer Fettphase und
- 5 bis 15 Gew.-% eines Emulgators, der folgendes umfasst:
• 10 bis 90 Gew.-%, vorzugsweise 10 bis 50 Gew.-% und ebenfalls bevorzugt 10 bis 40 Gew.-% eines Gemischs aus einem beliebigen Anteil eines Oleylglycosids mit einem Polymerisationsgrad zwischen 1 und 3 und eines Isostearylglycosids mit einem Polymerisationsgrad zwischen 1 und 3 und
• 90 bis 10 Gew.-%, vorzugsweise 90 bis 50 Gew.-% und ebenfalls bevorzugt 90 bis 60 Gew.-% eines Gemischs aus einem beliebigen Anteil von Oleylalkohol und Isostearylalkohol,
und gegebenenfalls:
- 0 bis 10 Gew.-% eines Coemulgators,
- 0 bis 5 Gew.-% eines Stabilisators.

2. Emulsionen nach Anspruch 1, **dadurch gekennzeichnet, dass** der vorstehend genannte Emulgator folgendes umfasst:
• 10 bis 25 Gew.-% eines Isostearylglycosids mit einem Polymerisationsgrad zwischen 1 und 3 und
• 90 bis 75 Gew.-% eines Isostearylalkohols.

3. Emulsionen nach Anspruch 1, **dadurch gekennzeichnet, dass** der vorstehend genannte Emulgator folgendes umfasst:
• 10 bis 25 Gew.-% eines Oleylglycosids mit einem Polymerisationsgrad zwischen 1 und 3 und
• 90 bis 75 Gew.-% eines Oleylalkohols.

4. Emulsionen nach einem der Ansprüche 1 bis 3, **dadurch gekennzeichnet, dass** die vorstehend genannte Fettphase aus einem oder mehreren Ölen besteht, die aus Mineralölen, insbesondere Paraffinölen, ausgewählt sind.

5. Emulsionen nach einem der Ansprüche 1 bis 4, **dadurch gekennzeichnet, dass** der vorstehend genannte Coemulgator aus einem Sorbitanester, vorzugsweise Sorbitanoleat, besteht.

6. Emulsionen nach einem der Ansprüche 1 bis 5, **dadurch gekennzeichnet, dass** der vorstehend genannte Stabilisator aus einem Polyethylenglycol-Dodecylglycol-Copolymer, wie das unter der Bezeichnung ELFACOS ST 9® vertriebene Produkt, Bienenwachs, Stearinsäure oder gehärtetem Ricinusöl ausgewählt ist.

7. Emulsionen nach einem der Ansprüche 1 bis 6, **dadurch gekennzeichnet, dass** sie als Coemulgator und Stabilisator ein Gemisch aus Bienenwachs, gehärtetem Ricinusöl, Stearinsäure und Sorbitanoleat, wie insbesondere das unter der Bezeichnung MONTANE® 481 vertriebene Produkt, enthalten.

8. Verwendung einer Zusammensetzung, die aus folgendem besteht:
• 10 bis 90 Gew.-%, vorzugsweise 10 bis 50 Gew.-% und ebenfalls bevorzugt 10 bis 40 Gew.-% eines Gemischs aus einem beliebigen Anteil eines Oleylglycosids mit einem Polymerisationsgrad zwischen 1 und 3 und eines Isostearylglycosids mit einem Polymerisationsgrad zwischen 1 und 3 und
• 90 bis 10 Gew.-%, vorzugsweise 90 bis 50 Gew.-% und ebenfalls bevorzugt 90 bis 60 Gew.-% eines Gemischs aus einem beliebigen Anteil von Oleylalkohol und Isostearylalkohol,
als Emulgator, der zur Herstellung einer stabilen Wasser-in-Öl-Emulsion bestimmt ist.

9. Verwendung nach Anspruch 8 einer Zusammensetzung, die aus folgendem besteht:
• 10 bis 25 Gew.-% eines Isostearylglycosids mit einem Polymerisationsgrad zwischen 1 und 3 und
• 90 bis 75 Gew.-% eines Isostearylalkohols,
als Emulgator, der zur Herstellung einer stabilen Wasser-in-Öl-Emulsion bestimmt ist.

10. Verwendung nach Anspruch 8 einer Zusammensetzung, die aus folgendem besteht:
• 10 bis 25 Gew.-% eines Oleylglycosids mit einem Polymerisationsgrad zwischen 1 und 3 und
• 90 bis 75 Gew.-% eines Oleylalkohols,
als Emulgator, der zur Herstellung einer stabilen Wasser-in-Öl-Emulsion bestimmt ist.
